(19) Europäisches Patentamt
European Patent Office
Office européen des brevets

(11) **EP 2 060 551 A1**

(12) **EUROPEAN PATENT APPLICATION**

(43) Date of publication:
**20.05.2009 Bulletin 2009/21**

(51) Int Cl.:
*C07C 2/86* *(2006.01)*  *C07C 9/16* *(2006.01)*
*C07C 11/02* *(2006.01)*  *B01J 29/06* *(2006.01)*

(21) Application number: **07254491.9**

(22) Date of filing: **16.11.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **BP p.l.c.**
**London SW1Y 4PD (GB)**

(72) Inventor: **The designation of the inventor has not yet been filed**

(74) Representative: **Oldroyd, Richard Duncan et al**
**BP International Ltd**
**Global Patents & Technology Law**
**(Central)**
**Chertsey Road**
**Sunbury-on-Thames, Middx TW16 7LN (GB)**

(54) **Process for producing triptane**

(57) A process for the production of triptane and/or triptene from methanol and/or one or more derivatives thereof and one or more olefins in the presence of a catalyst at a temperature in the range of from 150 to 400°C, in which the catalyst is a zeolite having Brønsted acidity and has a structure selected from:
i. Zeolites having frameworks comprising silicon and aluminium atoms and having a channel structure comprising a ring size of 12 or more non-oxygen framework atoms in 2 or 3 dimensions;

ii. Zeolites having frameworks comprising silicon and aluminium atoms and which have a framework ring comprising 12 or more non-oxygen atoms accessible on the external surface of the zeolite, and a channel structure in which all of the channels have a ring-size of less than 12 non-oxygen framework atoms,

and wherein zeolites of type (ii) comprise alkali metal where methanol is not a reactant.

EP 2 060 551 A1

**Description**

This invention relates to the production of triptane, more specifically to the production of triptane from methanol and/or one or more derivatives thereof and one or more olefins.

In gasoline formulations, branched hydrocarbons are generally favoured over linear hydrocarbons as they result in a fuel with a higher octane number. An example of a branched chain hydrocarbon desirable in gasoline formulations due to its high octane number is triptane (2,2,3-trimethylbutane), which can be used in unleaded aviation gasoline and unleaded motor gasoline, as described for example in WO 98/22556 and WO 99/49003.

Branched chain hydrocarbons may be synthesised by a number of routes, for example by homologation of methanol and/or dimethyl ether in the presence of a zinc halide catalyst, as described, for example in GB 1,547,955, US 2,492,984, US 3,969,427, US 4,059,646, US 4,059,647, US 4,249,031 and WO 02/70440.

In US 4,249,031 a process is described for the preparation of a hydrocarbon mixture by contacting one or more oxygen-containing organic compounds, such as methanol, with one or more zinc halides.

US 4,059,647 describes a process for the production of triptane by contacting methanol, dimethyl ether or mixtures thereof with zinc iodide.

Olefins can also be reacted with alcohols, in particular methanol, to produce hydrocarbons. For example, US 4,151,214 describes the use of zinc bromide or iodide catalysts in the production of triptane from methanol or dimethyl ether with an olefin.

However, a common problem associated with the use of halide-containing catalysts is that the halides can contribute to corrosion problems in plant equipment. Additionally, halides can contaminate the triptane product and needs to be removed before the triptane can be used as a fuel.

Zeolites have been reported as being able to produce alkanes from methanol or ethers, as described for example in US 3,894,106 and US 3,894,107. However, the products have high aromatic content, and nothing is taught about whether triptane is or could be created.

There remains a need for an alternative process for producing triptane.

According to a first aspect of the present invention, there is provided a process for the production of triptane and/or triptene from methanol and/or one or more derivatives thereof and one or more olefins, optionally in the presence of one or more further alcohols and/or derivatives thereof, which process comprises contacting a reaction composition comprising the methanol and/or one or more derivatives thereof, the one or more olefins, and optionally one or more further alcohols and/or derivatives thereof with a zeolite catalyst at a temperature in the range of from 150 to 400°C to produce triptane and/or triptene, characterised by the catalyst having Brønsted acidity and being selected from one or more of the following:

> i. Zeolites having frameworks comprising silicon and aluminium atoms and having a channel structure comprising a ring size of 12 or more non-oxygen framework atoms in 2 or 3 dimensions;
> ii. Alkali metal-containing zeolites having frameworks comprising silicon and aluminium atoms and which have a framework ring comprising 12 or more non-oxygen atoms accessible on the external surface of the zeolite, and a channel structure in which all of the channels have a ring-size of less than 12 non-oxygen framework atoms;

It has been found that crystalline zeolites, in particular aluminosilicate zeolites, can act as catalysts for the conversion of methanol and/or one or more derivatives thereof to triptane (2,2,3-trimethylbutane) and/or triptene (2,3,3-trimethylbut-1-ene) in the presence of one or more olefins. Triptane and triptene will henceforth be collectively referred to as triptyl compounds. The zeolite catalysts have at least some Brønsted acid character, and provide a combination of high triptyl yields and high fractions of triptyl compounds as a percentage of all $C_7$ hydrocarbons produced in the reaction. Triptyl fractions, as defined herein, are calculated as the percentage of triptyl compounds compared to all compounds that appear in a GC chromatogram with a retention time of greater than n-hexane up to and including the retention time of n-heptane using a boiling point separation column, such as a CP-Sil column or a DB-1 column).

As the crystalline aluminosilicate catalysts of the present invention do not comprise halide ions, then problems associated with halide contamination of the product and halide-induced corrosion in process equipment can be avoided.

Crystalline zeolites typically comprise an ordered inorganic oxide framework structure having a regular array of pores, often with enlarged cages where two or more pores intersect. Most commonly, the framework is principally aluminosilicate, comprising silicon and aluminium atoms. However, often zeolites can comprise other framework heteroatoms, for example phosphorus, gallium, titanium, germanium, iron and cobalt. Where present, these other framework heteroatoms are typically present at a level of up to 10 mole%, based on the total number of framework atoms.

For the zeolite to have Brønsted acidity, the overall charge on the framework must be negative, such that positive ions, in particular protons, are required to balance the charge. For example, the framework of aluminosilicate zeolites and silicoaluminophosphate zeolites is negatively charged, which negative charge can be balanced by protons to produce Brønsted acid sites. This type of Brønsted acidity is represented below in equation I.

$$[(SiO_2)_x(SiAlO_2)_y]^{y-} \cdot yH^+ \qquad \text{Equation I}$$

The framework negative charge can also be balanced by another cation, for example a transition metal or lanthanide ion, which itself can possess Brønsted acid character, for example by deprotonation of coordinated water molecules. This type of Brønsted acidity is represented below in equation II.

$$[(SiO_2)_x(SiAlO_2)_y]^{y-} \cdot (y/z)M^{z+} \cdot (H_2O)_a \cdot (OH^-) \cdot H^+ \qquad \text{Equation II}$$

Typically, the negative charges of the framework are counter-balanced by protons. The zeolite should have a sufficient number of acid sites to enable the acid-catalysed reaction of the methanol and/or derivatives thereof and one or more olefins to triptyl compounds to proceed. This can be conveniently expressed in terms of the framework silicon to aluminium mole ratio, which is typically 100 or less, for example 80 or less, such , as 50 or less.

Optionally, part of the negative charge of the zeolite framework can be counter-balanced by cations other than protons, for example ammonium ions or one or more alkali-metal ions, for example one or more of sodium, potassium, rubidium and caesium. Additionally, or alternatively, one or more alkaline-earth ions can be present, for example one or more of magnesium, calcium, strontium and barium. Additionally or alternatively, one or more p-block metal metals, transition metals and lanthanides can be present, for example one or more of lanthanum, cerium, silver, bismuth, copper, cobalt, indium, zinc, rhodium, platinum and palladium.

The number of acid sites in a zeolite can also be modified by treating the zeolite with organic silicon compounds or silicon compounds comprising halides, for example alkoxy silanes, alkyl silanes, silicon halides, or alkyl silicon halides. Specific examples include tetraethoxysilane, tetramethylsilane, silicon tetrachloride, and dimethyl dichloro silane. These react at proton sites, and hence by controlling the level of treatment a controlled quantity of acid sites can be blocked. Treated zeolites are typically treated by calcination in air at a temperature in the range of 400 to 600°C after treatment with the silicon compound.

The loading of the non-framework metals can be conveniently expressed in terms of a molar ratio compared to the element or elements of the zeolite responsible for imparting negative change to the zeolite framework. Such elements are henceforth referred to as T-elements for brevity. For example, in aluminosilicate zeolites, aluminium is the T-element. In gallo-aluminosilicate zeolites, both gallium and aluminium are T-elements. Thus, the loading can be expressed in terms of the total quantity of non-framework metal compared to the total quantity of T-element(s) in the zeolite.

On this basis, the loadings of non-framework metal, where present, are typically 1mol% or more compared to the total T-element content of the zeolite. More preferably, the loadings are in the range of from 1 to 10mol% of the total T-element content of the zeolite.

In the present invention, it has been found that zeolites of structure type (i) result in a combination of higher triptyl yields and higher triptyl fractions in the $C_7$ hydrocarbons compared to zeolites that do not adopt either the type (i) or type (ii) structures. For zeolites of structure type (ii), zeolites comprising one or more alkali-metals show the improved combination of higher triptyl yields and fractions compared to those that do not adopt either the type (i) or (ii) structures.

In the case of zeolite catalysts of type (i), the non-framework metal where present is preferably selected from one or more of copper, silver, and caesium. For type (ii) zeolite catalysts, the alkali-metal is preferably caesium.

The reaction that is catalysed is the production of triply compounds, i.e. triptane, triptene or mixtures thereof, from a reaction composition comprising methanol and/or one or more derivatives thereof, and one or more olefins. A derivative of methanol is a compound that can release methanol through a hydrolysis reaction. Examples of methanol derivatives include methyl esters, methyl ethers and methyl carbonates, specific examples being dimethyl ether and dimethyl carbonate. In a preferred embodiment, the reaction composition comprises methanol and/or dimethyl ether.

In a further embodiment, the reaction composition can comprise one or more further alcohols and/or derivatives thereof, an alcohol derivative being a compound that releases the corresponding alcohol through a hydrolysis reaction. Typically, when present, the one or more further alcohols and/or derivatives thereof are selected from one or more $C_2$ to $C_6$ alcohols and/or derivatives thereof, for example $C_2$ to $C_4$ alcohols and/or derivatives thereof, examples being ethanol, iso-propanol, n-propanol, iso-butanol, sec-butanol and n-butanol. The alcohol and/or derivative thereof can optionally be biologically derived. For example ethanol and butanol can be produced from the fermentation of biomass. This is advantageous, as the use of biologically-derived feedstocks can result in the triptane having a lower impact on atmospheric $CO_2$-emissions when combusted, because biomass ultimately derives from atmospheric $CO_2$.

The olefin is typically a $C_2$ to $C_6$ olefin, i.e. an olefin comprising 2 to 6 carbon atoms, such as ethene, propene, 1-butene, cis and trans 2-butene, isobutene, 2-methyl-2-butene and 2,3-dimethyl-2-butene. 2,3-dimethyl-2-butene is a particularly suitable olefin as triptyl compounds can be formed by the addition of a just single carbon atom at the olefin double bond. Pentenes or butenes can be a desirable constituent of the reaction composition, as they are potentially available in large quantities from refinery and petrochemical processes, being constituents of light hydrocarbon products from steam cracking or fluid catalytic cracking processes for example.

In a typical process, a reaction composition comprising methanol and/or one or more derivatives thereof, and the one or more olefins, optionally with one or more alcohols and/or derivatives thereof, are fed to a reactor in which the catalyst resides. A product stream is produced, comprising unconverted reactants, triptyl compounds and other by-products such as linear and branched alkanes and alkenes, cyclic hydrocarbons and aromatic compounds.

The reaction composition can optionally be diluted with an inert compound, such as nitrogen, argon or an alkane that is inert under the reaction conditions, such as methane or ethane.

The reaction composition can optionally comprise hydrogen, either in pure form or combined with an inert diluent, such as a hydrogen/nitrogen mixture. In this embodiment, the catalyst can optionally comprise metals active as hydrogenation catalysts, for example rhodium, platinum or palladium. The presence of hydrogen can help convert any triptene to triptane.

The mole ratio of methanol : olefin present in the reaction composition is suitably up to 100 : 1, and is preferably at least 2 : 1. Typically, the ratio is in the range of from 5 : 1 to 30 : 1. In this context, methanol relates to methanol present in the reaction composition and/or to methanol derivable from the one or more derivatives thereof present in the reaction composition. By methanol derivable from a methanol derivative is meant the number of methanol molecules that would be produced in the event of hydrolysis of the methanol derivative. For example, in the case of dimethyl ether (DME), two methanol molecules would result from hydrolysis. Similarly, a molecule of dimethyl carbonate (DMC) for example would also produce two molecules of methanol on hydrolysis. Thus, a DME or DMC : olefin mole ratio of 5 : 1 provides a methanol : olefin mole ratio of 10 : 1.

Typically, the reactions are conducted so that all reactants are in the gas-phase. Usually, the temperature is in the range of from 150 to 400°C, and preferably in the range of from 200 to 350°C, and even more preferably in the range of from 210°C to 300°C. The total gas hourly space velocity (GHSV) of the reactants over the catalyst is suitably up to 5000 $h^{-1}$, preferably 500 $h^{-1}$ or above, and more preferably 3000 $h^{-1}$ or less. Suitably, the GHSV is in the range of from 1000 to 3000 $h^{-1}$. In this case, the GHSV is given in units of mL gas, corrected to 0°C and 1 atm pressure, per mL catalyst per hour. The reaction pressure is typically in the range of from 1 bara (0.1 MPa) to 100 bara (10MPa).

In one embodiment of the invention, a mixture of carbon monoxide and hydrogen can be used to produce methanol and/or one or more derivatives thereof, and optionally further alcohols and/or derivatives thereof, in-situ within the reactor. This can be achieved by adapting the catalyst to comprise one or more additional components that are active for the production of methanol and/or derivatives thereof, and optionally additional alcohols and/or derivatives thereof from the carbon monoxide and hydrogen (henceforth referred to as an alcohols synthesis catalyst). In this way, the alcohols-containing reaction composition from which the triptyl compounds can be produced is generated directly from feedstocks such as syngas, which can be derived from natural gas, coal or biomass for example.

In this embodiment, the catalyst is suitably layered, such that the carbon monoxide and hydrogen-containing feedstock contacts the alcohols synthesis catalyst first, the so-formed methanol and/or one or more derivatives thereof and optionally further alcohols and/or derivatives thereof subsequently contact a layer of the zeolite catalyst of the present invention to form triptyl compounds.

In one embodiment of the invention, the alcohols synthesis catalyst is predominantly active for the formation of methanol and/or derivatives thereof, an example being a copper /zinc oxide/alumina (Cu/ZnO/Al$_2$O$_3$) catalyst.

In another embodiment, the catalyst is suitable for the production of mixed alcohols, such as a mixture of $C_1$ to $C_4$ alcohols and/or derivatives thereof, for example a cobalt-molydenum-sulphide catalyst (CoMoS).

The alcohols synthesis catalyst can, in a further embodiment, be mixed with an acidic catalyst, for example high surface area alumina, in order to increase the selectivity of the reaction towards ethers.

In an alternative embodiment, the process comprises two reactors, a first reactor having the alcohols synthesis catalyst, and a second reactor having the zeolite catalyst for triptyl formation. The carbon monoxide and hydrogen are fed to the first reactor to produce a product composition comprising methanol and/or one or more derivatives thereof, and optionally further alcohols and/or derivatives thereof, are produced, which then form at least part of the reaction composition for the triptyl-forming reaction. Optionally, before being fed to the second reactor, the product composition from the first reactor is treated to remove unwanted components, such as unreacted carbon monoxide and/or hydrogen, achieved, for example, through flash separation, and/or to remove by-products such as water, which can be achieved for example by distillation or by use of a selective molecular sieve absorbent. As optimum operating conditions of the alcohols forming and triptyl forming reactions are likely to be different, this separate reactor embodiment offers an advantage in enabling different operating conditions to be used for the two separate reactions. The one or more olefins are preferably fed to the second reactor to reduce their hydrogenation to corresponding alkanes, which would reduce thje potential yield of triptyl compounds.

Mixtures of carbon monoxide and hydrogen, where used, preferably have a H$_2$:CO mole ratio of 1:1 or above, for example in the range of 1:1 to 10:1, and more preferably in the range of from 1:1 to 6:1. Optionally, carbon dioxide can also be a constituent of the feedstock. Where present, the $H_2 : CO_x$ (i.e. $CO + CO_2$ mole ratio is suitably in the range of from 0.5:1 to 20:1, for example in the range of from 1:1 to 15:1, or 1:1 to 10:1.

Where the process comprises a mixed catalyst, and the alcohols forming and triptyl forming reactions occur within the same reactor, the temperature is preferably less than 310°C and is also preferably at least 240°C. More preferably, the temperature is in the range of from 250 to 300°C. The pressure is typically at least 10 bara (1 MPa) and typically at most 200 bara (20 MPa). Preferably, the pressure is at least 30 bara (3 MPa), and more preferably in the range of from 40 to 60 bara (4 to 6 MPa).

Where the alcohols-forming reaction and catalyst are in a separate reactor to the triptyl-forming reaction, the alcohols-production reaction typically operates at a temperature in the range of from 150 to 400°C, for example in the range of from 240 to 300°C, and a pressure in the range of from 10 to 150 bara (1 to 15 MPa), for example in the range of from 50 to 150 bara (5 to 15 MPa).

The alcohol and/or ether-containing product stream (including methanol and/or dimethyl ether) does not necessarily need to be treated to remove higher alcohols and/or ethers, as the higher alcohols and/or ethers can be useful components of the reaction composition for triptane formation.

The constituent components of the reaction composition may be pre-mixed before being fed to the reactor, or alternatively they may be fed separately to the reactor. The product stream resulting from the catalytic reaction or reactions is then treated so as to separate triptyl compounds from the unreacted reactants and by-products. Optionally, unreacted reactants and one or more by-products can be recycled to the reactor. Separation and purification typically utilises one or more units selected from flash separation vessels, distillation columns, decantation vessels and solid adsorbent beds.

It may not be necessary to completely isolate and purify the triptyl compounds. For example, in one embodiment, the product stream is treated so that triptyl compounds are present at a concentration sufficient for them to be blended with a gasoline fuel.

Optionally, the composition from the reaction is contacted with hydrogen in a separate reactor, in the presence of a hydrogenation catalyst such as a supported ruthenium, palladium or platinum catalyst, the support being for example carbon, alumina or silica. This converts olefins to the corresponding alkanes.

Using the catalysts of the present invention, it is possible to obtain a combination of triptyl yields of 0.8% or more and a triptyl fraction of greater than 5%. The triptyl yield is expressed as a percentage of all the carbon in the reaction composition, excluding any carbon associated with inert diluent. The triptyl fraction is expressed as a percentage of the $C_7$ hydrocarbons produced in the reaction. The triptyl fraction is determined experimentally by comparing the yield of triptyl compounds compared to all products appearing in a GC trace at a retention time exceeding that of n-hexane, up to and including n-heptane, when using a GC column that separates components on the basis of boiling point, such as a DB-1 or CP-Sil column.

Zeolites that appear to be most active towards triptyl formation are those having pores or cages with 12-membered rings or more. The ring size is defined by the number of non-oxygen atoms present in the ring. For example, in the case of an aluminosilicate zeolite having a 12-membered ring, the ring comprises a total of 12 silicon and aluminium atoms. If the zeolite is a silico-aluminophosphate, for example, then the ring comprises a total of 12 silicon, aluminium and phosphorus atoms. Non-framework atoms, for example charge balancing ions such as protons, alkali-metal ions or other positively charged cations which are not incorporated into the zeolite framework, are not considered to be part of the 12-membered ring.

It is believed that 12-membered rings are large enough to accommodate the reactant molecules and the necessary intermediates involved in triptyl formation. Although larger ring sizes would also be sufficiently large, for example having 14- or 16-membered rings, such zeolites are generally more unstable than those having 12-membered rings.

There are two types of zeolite structure that are able to provide a combination of high triptyl yields and also high triptyl fractions in the $C_7$ hydrocarbons. These are henceforth labelled zeolite types (i) and (ii) for convenience.

Type (i) zeolites comprise a 2 or 3-dimensional network of pores, the ring size of the pores being 12 or more non-oxygen framework atoms in at least 2 dimensions. Zeolites in this category include zeolites of the FAU, BEA or EMT structures. Full details of zeolite structures can be found in the Atlas of Zeolite Structure Types, available from the International Zeolite Association.

In one embodiment of the invention, the zeolite has the faujasite (FAU) structure, such as zeolite X or Y, which comprises channels with 12-membered ring channel openings, with a diameter of around 7.4 Å. The channel structure is 3-dimensional, and has cages with a diameter of around 12.7 Å. Typical framework Si/Al molar ratios of zeolite X are up to 2. For zeolite Y, the ratio is typically above 2. Zeolite X has been shown to be highly active towards triptyl production.

Zeolite USY, short for "Ultra-Stable" Y, or zeolite VUSY, short for "Very Ultra-Stable" Y, are produced when zeolite Y is subjected to a dealumination treatment, typically using high temperature steam treatment. This results in aluminium being extracted from the framework, resulting in so called extra-framework alumina particles and leaving a disrupted zeolite framework structure. At high levels of dealumination, for example in the case of VUSY, the zeolite is not so active towards triptyl formation, and typically only exhibits significant triptyl activity at temperatures above 275°C, for example at 350°C or above. This is possibly a result of the number and density of framework acid sites being reduced to too great an extent for reaction to occur. However, it is believed that a lesser degree of dealumination may be advantageous because a disrupted framework can allow improved access to a greater number of acidic sites of the zeolite. Therefore, in dealuminated zeolites, a balance must be made between improving access to the acidic sites of the zeolite, and maintaining sufficient framework acid site density to enable the triptyl-forming reaction to occur.

Another zeolite structure having 12-membered ring channel openings is the BEA structure, as exhibited by zeolite Beta, which also has a 3-dimensional channel structure. Si/Al molar ratios of aluminosilicate zeolite Beta are typically from 5 to 100, as described in US 3,308,069.

Yet another zeolite structure having 12-membered rings and a 3-dimensional pore structure is the EMT structure, as exhibited for example by zeolite ZSM-3, as described in US 3,415,736, and by zeolite ZSM-20 as described in US 3,972,983.

Zeolites of structure type (ii) have pores with a ring size of less than 12 non-oxygen framework atoms in all dimensions. However, the framework structure comprises features having 12 or more-membered rings available at the zeolite surface.

An example of such a structure is the MWW structure, which has a two-dimensional pore structure, in which all the pores in the network have a 10-membered ring at their narrowest portion. However, the channels intersect at cylindrical cages formed of 12-membered rings, the cage dimensions being about 7.1Å diameter across the short axis and about 18.2Å in length. Thus, although access to the 12-membered ring cages is restricted by the 10-membered ring openings, the external surfaces of the crystals comprise opened cages, thus making the 12-membered rings accessible to reactants. MCM-22, described in US 4,992,615, is an example of an aluminosilicate zeolite with this structure, typically having a Si/Al ratio of 5 or more. In MCM-22, the cages appear as cups or pockets on the crystal surface, having a diameter of about 7.1Å and a depth of about 7.0Å.

Other examples of zeolites suitable for use in the present invention are delaminated zeolites. ITQ-2 is an example of a delaminated zeolite. The ITQ-2 framework is based on that of the layered MCM-22 (MWW) structure, of the structure type (ii), except that condensation of the MCM-22 layers at the framework cages is disrupted during the synthesis, resulting in a disordered layered structure and an increased proportion of 12-membered ring cages open and accessible to reactants at the external crystal surface. The synthesis and structure of ITQ-2 is described in WO 01/21562.

Without being bound by any theory, it is thought that the advantageous features of the zeolite catalysts used in the process of the present invention can be attributed to the need for a relatively large pocket, cage or channel which can accommodate the triptyl molecules when they are formed, while reducing any restriction that may prevent the triptyl molecules diffusing away from the catalyst. Thus, in the case of MCM-22 and ITQ-2 for example (which adopt the MWW structure), the surface 12-membered ring pockets allow the triptyl molecules to form, but the small channels having 10-membered ring restrictions prevents their diffusion into the zeolite framework. In the case of zeolite Y or Beta for example, with FAU and BEA structure types respectively, although the 12-membered ring channels allow access to the interior of the zeolite structure, diffusion is not inhibited as the interconnecting channel structure allows a facile route out of the catalyst interior.

In a second aspect of the present invention, it has been found that when methanol is a constituent of the reaction composition, then protonated and other metal-loaded forms of zeolite having structure type (ii) also exhibit both improved yields and increased triptyl fractions compared to zeolites that do not adopt zeolite structure types (i) and (ii).

Thus, according to a second aspect of the present invention, there is provided a process for the production of triptane and/or triptene from methanol and one or more olefins, optionally in the presence of one or more further alcohols and/or derivatives thereof, which process comprises contacting a reaction composition comprising the methanol, the one or more olefins, and optionally the one or more further alcohols and/or derivatives thereof with a catalyst active for the production of triptane and/or triptene at a temperature in the range of from 150 to 400°C to produce triptane and/or triptene, characterised by the catalyst having Brønsted acidity and being selected from one or more zeolites having frameworks comprising silicon and aluminium atoms and which have a framework ring comprising 12 or more non-oxygen-atoms accessible on the surface of the zeolite and a pore structure in which all of the channels have a ring-size of less than 12 non-oxygen atoms.

According to this second aspect of the invention, the zeolites of type (ii) are active where methanol is present in the feedstock, whether fully protonated or metal-loaded, and are able to provide both improved triptyl yields and triptyl fractions compared to zeolites that do not adopt structure types (i) and (ii). The structure type (ii), as stated above, is exemplified by the MWW structure, and is adopted by zeolites such as MCM-22 and ITQ-2. Metal cations where present are preferably selected from one or more of silver and copper (in addition to alkali metals such as caesium), with loadings of 1mol% or more, preferably in the range of from 1 to 10 mol%, based on the T-element content of the zeolite.

In both aspects of the present invention, the zeolite catalysts of the present invention are capable of providing triptyl yields of 0.8% or more based on the total carbon in the feedstock (excluding any carbon associated with inert diluent), and also triptyl fractions of greater than 5%, expressed as a percentage of all $C_7$ compounds produced in the reaction.

Experimentally, the triptyl fraction is determined by calculating the percentage yield of triptyl compared to all products appearing in a GC trace at a retention time exceeding that of n-hexane, up to and including n-heptane, when using a GC column that separates components on the basis of boiling point, such as a DB-1 or CP-Sil column.

The invention will now be illustrated by the following non-limiting examples, and with reference to the Figures in which;

Figure 1 is a graph showing the yield of triptyl compounds for a series of zeolites in their protonated form for reaction of a nitrogen-diluted dimethyl ether and 2, 3-dimethyl-2- _ butene reaction composition at 275°C, 1 bara pressure and a GHSV of 2000 h$^{-1}$.

Figure 2 is a graph showing the triptyl fraction as a percentage of $C_7$ hydrocarbons formed in the same experiments as shown in Figure 1.

Figure 3 is a graph showing triptyl yields for zeolite catalysts using a nitrogen-diluted dimethyl ether and 2,3-dimethyl-2-butene feedstock.

Catalytic experiments were conducted using a multi-reactor block comprising 16 single pass tube reactors each of 2mm internal diameter. Catalyst particles of 50 to 200 micrometers in diameter were loaded into the reactors. Each reactor had a dedicated and separate supply of reactants and inert diluent and separate liquid collection vessels for collection of high-boiling components that could cause fouling of the gas analysis equipment. Gas-phase material coming out of the reactors were fed to a common GC apparatus for analysis.

Zeolites were either purchased from commercial sources, or were prepared using literature methods where not commercially available. They were converted to the ammonium ion form by a triple ion-exchange treatment with 0.2M ammonium nitrate solution, the material being decanted and water-washed between each ion exchange treatment. The resulting material was left to dry at 50°C, and calcined in air at 400°C. Before use, the ammonium-exchanged material was heated under nitrogen at 400°C to convert to the acid form. Reported activities relate to the first 400 minutes of the reaction, unless otherwise stated.

Metal-loaded zeolites were prepared by incipient wetness impregnation, by evaporation a suspension of the zeolite in an aqueous solution of relevant soluble metal salts to dryness at a temperature of 50-60°C. The material was then dried at 110°C for 1 hour, and calcined in air overnight at 500°C.

With the exception of zeolite X, all zeolites had a silicon : aluminium mole ratio within the range of greater than 2 and up to 50. MCM-22 samples had an Si:Al mole ratio of 25. Zeolite X had an Si:Al mole ratio of 1.2. In all experiments, the yields of triptyl compounds (where observed) under reaction conditions were typically up to 15%. The main other hydrocarbon products observed in the gas-phase were non-triptyl hydrocarbons having 4 to 8 carbon atoms, with some oligomeric hydrocarbons with more than 8 carbon atoms also being apparent. Liquid-phase product was also collected, and comprised heavier compounds such as methylated aromatics.

Experiment 1

The feedstock was a mixture of methanol and 2,3-dimethyl-2-butene (2,3-DMB) in a 20:1 molar ratio, diluted in nitrogen at a nitrogen : (methanol + 2,3-DMB) molar ratio of 4:1. The feedstock was fed over the catalyst at a pressure of 1 bara, a temperature of 275°C, and a total GHSV of 2000 h$^{-1}$. The following aluminosilicate zeolites in their protonated form were tested: Beta, ZSM-5, MCM-22, X, Y and ZSM-12. Ag, Cu and Cs-loaded analogues were also studied, with loadings ranging from 1 to 10% of the total T-element content of the zeolite. In these examples, aluminium was the only T-element. ZSM-5 and ZSM-12 exhibited at least some activity towards triptyl formation. However, only fully protonated and metal-loaded zeolites Beta, X, and Y and MCM-22 showed activity sufficient to give triptyl yields of 0.8% or more and triptyl fractions of greater than 5% of the $C_7$ hydrocarbons formed.

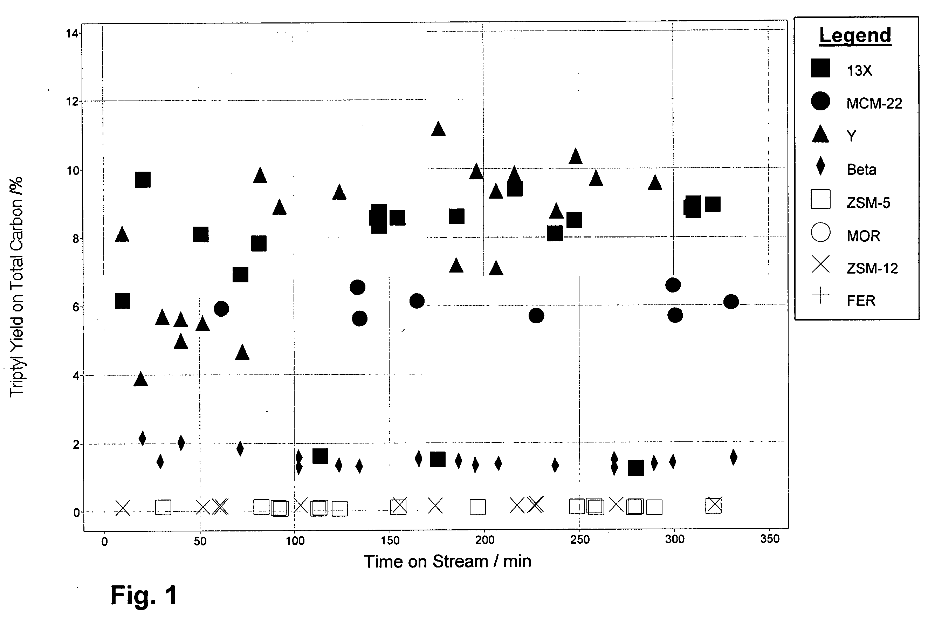

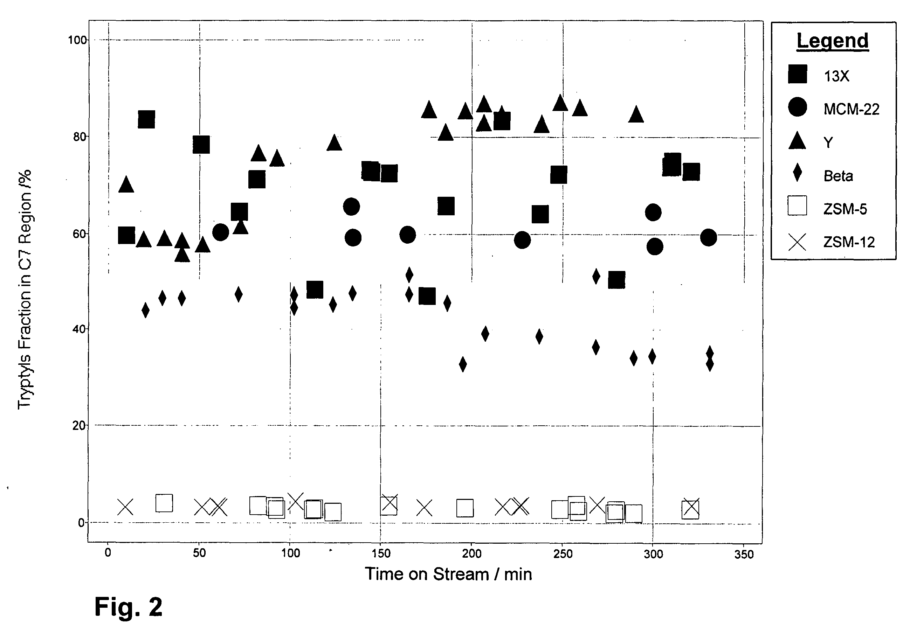

Triptyl yields are listed in Table 1 and illustrated in Figure 1. Triptyl fractions are listed in Table 2 and illustrated in Figure 2.

Experiment 2

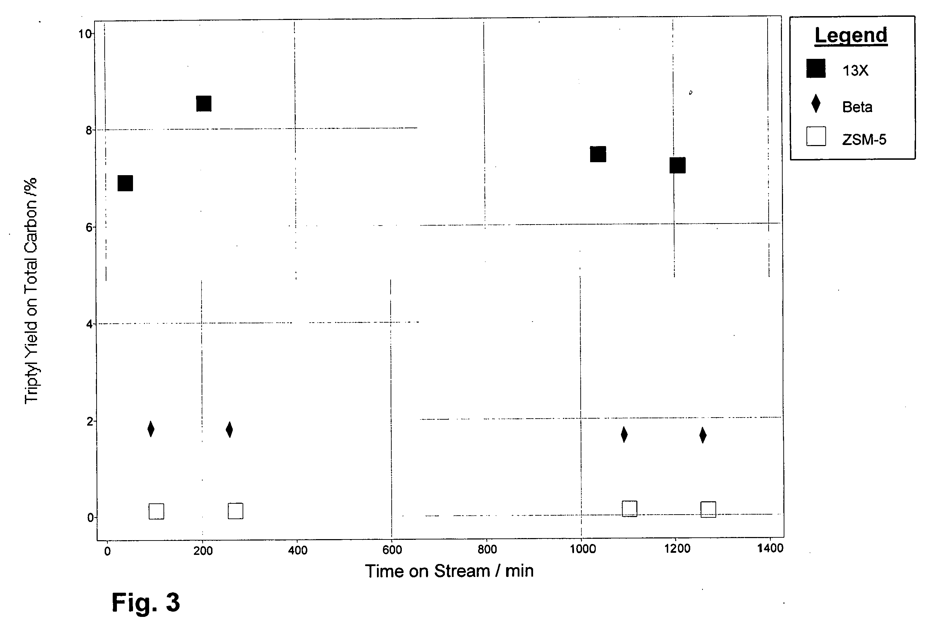

This was conducted using a dimethyl ether (DME) and 2,3-DMB feedstock diluted in nitrogen. The DME : 2,3-DMB mole ratio was 10:1. The nitrogen to (DME + 2,3-DMB) mole ratio was 4 : 1. Total GHSV was 2000 h$^{-1}$, and reaction temperature was 275°C. The zeolites tested were the same as in Experiment 1. Triptyl yields are tabulated in Table 3, and plotted in Figure 3. Triptyl fractions are listed in Table 4. Full protonated forms of zeolites Beta, X and Y, and the metal-loaded analogues, gave triptyl yields of 0.8% or more and triptyl fractions of greater than 5%. Of the MCM-22 catalysts studied, only the Cs-loaded material provided a triptyl yield of 0.8% or more and a triptyl fraction of greater than 5%.

Table 1 : Tripyl Yields for zeolite catalysts using methanol/2,3-DMB Feedstock

| | | Triptyl Yield (%)[a] | | | | | | | | |
|---|---|---|---|---|---|---|---|---|---|---|
| | | Cu | | | Ag | | | Cs | | |
| Zeolite | H+ | 1%[b] | 2.5%[b] | 10%[b] | 1%[b] | 2.5%[b] | 10%[b] | 1%[b] | 2.5%[b] | 10%[b] |
| MCM-22 | 5.8 | 6.6 | - | - | - | 5.7 | - | 6.1 | - | - |
| X | 1.4 | 8.7 | 8.2 | 8.6 | 8.9 | 8.8 | 7.5 | 3.9 | 9.2 | 8.8 |
| Y | 7.7 | 7.8 | 9.5 | 6.7 | 6.1 | 7.5 | 5.5 | 9.3 | 10.1 | 9.7 |
| Beta | 1.6 | 1.8 | 1.4 | 1.4 | 1.4 | 1.7 | 1.5 | 2.7 | 1.4 | 1.5 |
| ZSM-5 | 0.1 | 0.1 | - | 0.2 | 0.1 | - | 0.1 | 0.1 | - | 0.1 |
| ZSM-12 | 0.2 | 0.2 | - | 0.2 | - | - | 0.2 | 0.2 | - | 0.2 |

[a]percentage of tripyls based on total carbon in feedstock, average over 400 minutes.
[b]expressed as a percentage of the total T-element content of the zeolite.

Table 2 : Triptyl Fractions for zeolite catalysts using methanol/2,3-DMB Feedstock

| | | Triptyl Fraction (%)[a] | | | | | | | | |
|---|---|---|---|---|---|---|---|---|---|---|
| | | Cu | | | Ag | | | Cs | | |
| Zeolite | H+ | 1%[b] | 2.5%[b] | 10%[b] | 1%[b] | 2.5%[b] | 10%[b] | 1%[b] | 2.5%[b] | 10%[b] |
| MCM-22 | 59.7 | 65.1 | - | - | - | 58.5 | - | 59.7 | - | - |
| X | 49.6 | 73.5 | 71.8 | 73.9 | 73.6 | 72.8 | 64.5 | 53.4 | 74.7 | 80.9 |
| Y | 71.2 | 72.2 | 81.8 | 72.2 | 69.4 | 72.7 | 69.9 | 80.9 | 81.8 | 78.0 |
| Beta | 43.0 | 44.8 | 40.6 | 39.6 | 39.7 | 42.9 | 42.3 | 41.2 | 49.3 | 41.4 |
| ZSM-5 | 3.5 | 2.8 | - | 3.8 | 3.1 | - | 2.4 | 3.1 | - | 3.8 |
| ZSM-12 | 3.4 | 3.5 | - | 3.5 | - | - | 3.5 | 4.1 | - | 4.2 |

[a]facrtion of triptyls based on $C^7$ hydrocarbon products, average over 400 minutes.
[b]expressed as a percentage of the total T-element content of the zeolite.

Table 3: triptyl Yields for zeolite catalysts using Dimethyl Ether/2,3-DMB feedstock.

| | | Triptyl Yield (%)[a] | | | | | | | | |
|---|---|---|---|---|---|---|---|---|---|---|
| | | Cu | | | Ag | | | Cs | | |
| Zeolite | H+ | 1%[b] | 2.5%[b] | 10%[b] | 1%[b] | 2.5%[b] | 10%[b] | 1%[b] | 2.5%[b] | 10%[b] |
| MCM-22 | 0.2 | 0.2 | - | - | - | 0.2 | - | 1.2 | - | - |
| X | 7.7 | 6.5 | 4.6 | 1.5 | 6.3 | 4.5 | 1.0 | 8.1 | 8.7 | 7.0 |
| Y | 5.1 | 3.7 | 3.4 | 3.6 | 4.7 | 3.5 | 0.8 | 5.7 | 4.8 | 5.2 |
| Beta | 2.2 | 1.0 | 1.7 | 6.2 | 1.9 | 1.6 | 2.0 | 2.3 | 2.2 | 2.0 |
| ZSM-5 | 0.2 | 0.2 | - | 0.2 | 0.2 | - | 0.2 | 0.2 | - | 0.3 |
| ZSM-12 | 0.3 | 0.2 | - | 0.3 | - | - | 0.5 | 0.2 | - | 0.2 |

[a]percentage of triptyls based on total carbon in feedstock, averaged over 400 minutes.
[b]expressed as a percentage of the total T-element content of the zeolite.

# EP 2 060 551 A1

Table 4 : Triptyl Fractions for zeolite catalysts using Dimethyl Ether/2,3-DMB feedstock.

| Zeolite | H+ | Triptyl Fraction (%)[a] | | | | | | | | |
|---|---|---|---|---|---|---|---|---|---|---|
| | | Cu | | | Ag | | | Cs | | |
| | | 1%[b] | 2.5%[b] | 10%[b] | 1%[b] | 2.5%[b] | 10%[b] | 1%[b] | 2.5%[b] | 10%[b] |
| MCM-22 | 33.9 | 1.1 | - | - | - | 24.6 | - | 18.7 | - | - |
| X | 71.3 | 74.9 | 27.7 | 89.2 | 74.0 | 78.8 | 91.6 | 68.0 | 91.3 | 66.5 |
| Y | 71.1 | 22.9 | 19.8 | 65.3 | 70.7 | 44.7 | 68.4 | 67.9 | 67.4 | 74.4 |
| Beta | 51.9 | 30.5 | 50.2 | 83.7 | 49.8 | 48.3 | 51.4 | 92.3 | 53.8 | 52.4 |
| ZSM-5 | 1.0 | 49.4 | - | 4.1 | 31.6 | - | 45.5 | 48.8 | - | 9.8 |
| ZSM-12 | 1.7 | 45.6 | - | 1.8 | - | - | 25.4 | 58.6 | - | 1.3 |

[a]fraction of triptyls based on $C_7$ hydrocarbon products, averaged over 400 minutes.
[b]expressed as a percentage of the total 6-element content of the zeolite.

**Claims**

1. According to a first aspect of the present invention, there is provided a process for the production of triptane and/or triptene from methanol and/or one or more derivatives thereof, and one or more olefins, optionally in the presence of one or more further alcohols and/or derivatives thereof, which process comprises contacting a reaction composition comprising the methanol and/or one or more derivatives thereof, the one or more olefins, and optionally one or more further alcohols and/or derivatives thereof with a zeolite catalyst at a temperature in the range of from 150 to 400°C to produce triptane and/or triptene, **characterised by** the catalyst having Brønsted acidity and being selected from one or more of the following:

    i. Zeolites having frameworks comprising silicon and aluminium atoms and having a channel structure comprising a ring size of 12 or more non-oxygen framework atoms in 2 or 3 dimensions;
    ii. Alkali metal containing zeolites having frameworks comprising silicon and aluminium atoms and which have a framework ring comprising 12 or more non-oxygen atoms accessible on the external surface of the zeolite, and a channel structure in which all of the channels have a ring-size of less than 12 non-oxygen framework atoms;

2. A process as claimed in claim 1, in which the zeolite catalyst is selected from (i) and has the FAU, or BEA structure.

3. A process as claimed in claim 3, in which the zeolite catalyst is selected from zeolite Y, zeolite X, and zeolite Beta.

4. A process as claimed in claim 4, in which the zeolite catalyst comprises non-framework metal ions.

5. A process as claimed in claim 5, in which the non-framework metal ions are present at a loading of 1 to 10 mol%, based on the negative charge of the framework.

6. A process as claimed in claim 6, in which the non-framework metal ions are selected from one or more of Ag, Ba, Bi, Ca, Ce, Cu, Co, Cs, In, La, Li, Rh, Pd, Pt and Zn.

7. A process as claimed in claim 7, in which the non-framework metal ions are selected from one or more of Ag, Cu and Cs.

8. A process as claimed in claim 1 or claim 2, in which the zeolite catalyst is selected from (ii) and has the MWW structure.

9. A process as claimed in claim 9, in which the zeolite catalyst is MCM-22.

10. A process as claimed in any one of claims 1 to 9, in which the zeolite catalyst is an aluminosilicate.

11. A process as claimed in any one of claims 1 to 10, in which the zeolite catalyst comprises heteroatoms in the framework selected from one or more of phosphorus, gallium, titanium, germanium, vanadium, iron and cobalt at a loading of up to 10mol% based on the number of framework atoms.

**12.** A process as claimed in claim 8 or claim 9, in which the alkali metal is Cs and is present at a loading of 1mol% or more based on the T-element content of the zeolite.

**13.** A process as claimed in any one of claims 1 to 12, in which the reaction composition comprises methanol and/or dimethyl ether.

**14.** A process for the production of triptane and/or triptene from methanol and one or more olefins, optionally in the presence of one or more alcohols and/or derivatives thereof, which process comprises contacting a reaction composition comprising the methanol, the one or more olefins, and optionally the one or more further alcohols and/or derivatives thereof with a zeolite catalyst active for the production of triptane and/or triptene at a temperature in the range of from 150 to 400°C to produce triptane and/or triptene, **characterised by** the catalyst having Brønsted acidity and being selected from one or more zeolites having frameworks comprising silicon and aluminium atoms and which have a framework ring comprising 12 or more non-oxygen-atoms accessible on the surface of the zeolite and a pore structure in which all of the channels have a ring-size of less than 12 non-oxygen atoms.

**15.** A process as claimed in claim 14, in which the zeolite has the MWW structure.

**16.** A process as claimed in claim 15, in which the zeolite is MCM-22.

**17.** A process as claimed in claim 16, in which non-framework metal ions are present at a loading of 1 to 10 mol% of the total T-element content of the zeolite catalyst.

**18.** A process as claimed in claim 17, in which the non-framework metal ions are selected from one or more of Ag, Ba, Bi, Ca, Ce, Cu, Co, In, La, Li, Rh, Pd, Pt and Zn.

**19.** A process as claimed in claim 18, in which the non-framework metal ions are selected from one or more of Ag and Cu.

**20.** A process as claimed in any one of claims 1 to 19, in which the reaction composition comprises one or more further oxygen-containing carbon compounds selected from $C_2$ to $C_6$ alcohols and/or derivatives thereof.

**21.** A process as claimed in any one of claims 1 to 20, in which the olefins are selected from $C_2$ to $C_6$ olefins.

**22.** A process as claimed in claim 21, in which the olefin is 2,3-dimethyl-2-butene.

**23.** A process as claimed in any one of claims 1 to 22, in which the process is operated at a temperature in the range of from 200 to 350°C, and a GHSV of in the range of 5000 $h^{-1}$ or below.

**24.** A process as claimed in any one of claims 1 to 23, in which the GHSV is 500 $h^{-1}$ or above.

**25.** A process as claimed in any one of claims 1 to 24, in which the triptyl yield is 0.8% or more, and the triptyl fraction is greater than 5%.

EP 2 060 551 A1

**Fig. 1**

Fig. 2

Fig. 3

**European Patent Office**

## EUROPEAN SEARCH REPORT

Application Number

EP 07 25 4491

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 3 082 272 A (LONG ROBERT B) 19 March 1963 (1963-03-19) * claims 3,5,8; example 4 * ----- | 1,14 | INV. C07C2/86 C07C9/16 C07C11/02 B01J29/06 |
| Y | JP 62 121787 A (MOBIL OIL CORP) 3 June 1987 (1987-06-03) * abstract * ----- | 1,14 | |
| Y | EP 0 485 145 A (BRITISH PETROLEUM CO PLC [GB]) 13 May 1992 (1992-05-13) * the whole document * ----- | 1,14 | |
| E | EP 1 900 714 A (BP OIL INT [GB]) 19 March 2008 (2008-03-19) * the whole document * ----- | 1-25 | |
| D,A | US 4 151 214 A (KIM LEO [US] ET AL) 24 April 1979 (1979-04-24) * the whole document * ----- | 1-25 | |
| | | | TECHNICAL FIELDS SEARCHED (IPC) |
| | | | C07C B01J |

The present search report has been drawn up for all claims

| Place of search | Date of completion of the search | Examiner |
|---|---|---|
| Munich | 23 April 2008 | Breimaier, Waltraud |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 07 25 4491

This annex lists the patent family members relating to the patent documents cited in the above-mentioned European search report.
The members are as contained in the European Patent Office EDP file on
The European Patent Office is in no way liable for these particulars which are merely given for the purpose of information.

23-04-2008

| Patent document cited in search report | | Publication date | Patent family member(s) | | Publication date |
|---|---|---|---|---|---|
| US 3082272 | A | 19-03-1963 | NONE | | |
| JP 62121787 | A | 03-06-1987 | ZA | 8607243 A | 27-04-1988 |
| EP 0485145 | A | 13-05-1992 | AU | 648603 B2 | 28-04-1994 |
| | | | AU | 8708291 A | 14-05-1992 |
| | | | CA | 2055074 A1 | 09-05-1992 |
| | | | CN | 1061214 A | 20-05-1992 |
| | | | FI | 915277 A | 09-05-1992 |
| | | | JP | 4273831 A | 30-09-1992 |
| | | | NO | 914360 A | 11-05-1992 |
| | | | NZ | 240516 A | 23-12-1993 |
| | | | US | 5210364 A | 11-05-1993 |
| | | | ZA | 9108844 A | 07-05-1993 |
| EP 1900714 | A | 19-03-2008 | WO | 2008032015 A1 | 20-03-2008 |
| US 4151214 | A | 24-04-1979 | NONE | | |

EPO FORM P0459

For more details about this annex : see Official Journal of the European Patent Office, No. 12/82

**EP 2 060 551 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9822556 A
- WO 9949003 A
- GB 1547955 A
- US 2492984 A
- US 3969427 A
- US 4059646 A
- US 4059647 A
- US 4249031 A
- WO 0270440 A
- US 4151214 A
- US 3894106 A
- US 3894107 A
- US 3308069 A
- US 3415736 A
- US 3972983 A
- US 4992615 A
- WO 0121562 A